# EUROPEAN PATENT SPECIFICATION

(11) **EP 0 589 407 B1**
(45) Date of publication and mention of the grant of the patent: **30.08.1995**
(21) Application number: 93115164.1
(22) Date of filing: 21.09.1993
(51) Int. Cl.: A61K 7/48, A61K 7/06, A61K 7/50, A61K 7/08

(54) **Liquid personal cleansing composition**
Flüssiges persönliches Reinigungsmittel
Composition liquide de nettoyage personnel

(30) Priority: 22.09.1992 DE 4231661
(43) Date of publication of application: 30.03.1994
(73) Proprietor: Kao Corporation, Chuo-Ku Tokyo 103 (JP)
(72) Inventor: Schupp, Bettina, D-64297 Darmstadt (DE); Onitsuka, Satoshi, D-64295 Darmstadt (DE); Kameda, Takuo, D-64342 Seeheim-Jugenheim (DE)
(74) Representative: Patentanwälte Zellentin & Partner

(56) References cited:
- EP-A- 0 070 074
- DE-A- 4 129 124
- DE-U- 9 104 777
- DATABASE WPI Week 9338, Derwent Publications Ltd., London, GB; AN 93-297778
- DATABASE WPI Week 8636, Derwent Publications Ltd., London, GB; AN 86-236766

## Description

This invention refers to a liquid personal or body cleansing composition on an aqueous basis, especially for use as bubble bath, shower gel or hair shampoo, which has a stable lathering power and conditioning performance on skin and hair when applied with water, and particularly if applied on a dry hair type providing softness and pliability.

According to the invention, this problem is solved by adding to the aqueous body cleansing composition containing about 5 to about 50% by weight of at least one anionic surfactant, triglycerol and (or) diglycerol in a proportion of about 0.1 to about 10% by weight, preferably 0.25 to 7.5% by weight, most preferred about 0.5 to 5% by wt. calculated to the total composition.

Further to the improved conditioning and hair or skin smoothing properties, an admixture of triglycerol also allows to produce liquid compositions which exhibit improved transparency.

As suitable anion-active surfactants for the body cleansing compositions according to the invention, those generally used in such products should be named, e.g., the known C₁₀-C₁₈-alkyl sulfates and particularly the corresponding ether sulfates, e.g., C₁₂-C₁₄-alkyl ether sulfate, laurylether sulfate, especially having 1 to 4 ethylene oxide groups within the molecule, furtheron monoglyceride sulfates, fatty acid amide sulfates obtained by ethoxylation and subsequent sulfatation of fatty acid alkanol amides, alkylether carboxylic acids and the alkali salts thereof as well as long-chain mono- and dialkylphosphate salts which are skin compatible detergents.

Another preferred group of anionic surfactants are long-chain acyl sarcosinates or the alkali salts thereof, e.g. sodium lauroyl sarcosinate.

Within the scope of the invention, alpha-olefin sulfonates or their salts are of course also suitable anionic surfactants, particularly alkali salts of sulfosuccinic acid semiesters, e.g., the disodium salt of monooctyl sulfosuccinate and alkali salts of long-chain monoalkyl ethoxysulfosuccinates.

It is especially advisable to use mixtures of several anionic surfactants, e.g., a mixture of an alpha-olefin sulfonate and a sulfocuccinate, preferably in a proportion of 1 : 3 to 3 : 1.

In admixture with other anionic surfactants protein fatty acid condensation products of known structure per se may also be used, especially in a proportion between about 0.5 and 5%, preferably 1 to 3% by weight of the total liquid body cleansing composition.

A summary of the anionic surfactants used in liquid body cleansing compositions is also available in the monography of K.Schrader, "Grundlagen und Rezepturen der Kosmetika", 2nd Edition (1989, Hüthig Buchverlag), pp. 683 to 691.

For the liquid body cleansing compositions, the preferred quantity of anionic surfactants is in the range of about 5 and about 35% by weight, particularly from about 7.5 to about 25% by wt., most preferred from about 10 to about 20% by weight, calculated to the total composition of the product.

According to a preferred embodiment of the invention, the liquid body cleansing composition additionally contains, calculated to the total composition, about 1 to about 15% by weight, preferably about 2.5 to about 10% by weight of at least one nonionic surfactant.

In this context, a preferred nonionic surfactant belongs to the group of alkyl polyglucosides of the general formula

R-O-(R¹O)ₙ-Zₓ,

wherein R denotes an alkyl group having 8 to 18 carbon atoms, R¹ denotes an ethylene or propylene group, Z denotes a saccharide residue having 5 to 6 carbon atoms, n stands for a number from zero to ten, and x for a number between 1 and 2.5.

These alkyl polyglucosides have recently become known specially as skin-compatible compounds which excellently improve the lathering property in liquid washing and body cleansing compositions.

Although alkyl polyglucosides of the above general formula are preferred as nonionic surfactants in the compositions according to the invention, it is also possible to use further nonionic surfactants instead of, or rather together with the same. As such amine oxides are particularly suitable, e.g. lauryldimethyl amine oxide or coconut di(hydroxyethyl) amine oxide.

Further nonionic surfactant ingredients are also long-chain fatty acid mono- and dialkanolamides, e.g., coconut fatty acid monoethanolamide and myristic fatty acid monoethanolamide, which may also be used as foam boosters.

Other nonionic surfactants, e.g., are the various sorbitan esters such as polyethylene glycolsorbitan stearic acid esters, fatty acid polyglycol esters or mixed polycondensates of ethylene oxide and propylene oxide as they are on the market, e.g., under the trade name "Pluronics".

The compositions according to the invention may also contain amphoteric surfactants, preferably in a proportion of 0.5 to about 5% by wt., calculated to the total composition. In this context particularly the various known betaines are mentioned such as fatty acid amidoalkyl betaines and sulfobetaines, e.g., laurylhydroxy sulfobetaine; also long-chain alkylamino acids have proved suitable.

A list of suitable amphoteric surfactants is included in Schrader, l.c., pp. 598 to 600.

Mixtures of anion-active surfactants and alkyl polyglucosides, which are the preferred nonionic surfactants within the scope of the invention, and their use in liquid body cleansing compositions are known per se, e.g. from European Patent Application No.70,074.

The alkyl polyglucosides described therein are principally also suitable within the scope of the present invention; also suitable are mixtures of sulfosuccinates and alkyl polyglucosides known from European Patent No.358,216.

The liquid body cleansing compositions according to the invention may obviously comprise all further substances usually employed in these compositions.

As such are mentioned: complexing and colouring agents, preservatives, pH-regulators, viscosity regulators such as inorganic salts as far as they are not anyway part of the initial surfactant mixture, fragrances, pearl-gloss agents, thickening agents, humectants, vegetable or animal oils such as jojoba oil, etc.

A list of these additives may also be found in Schrader, l.c., for bubble bath and shower gel additives on pp. 596 to 620, and for shampoos on pp. 695 to 722.

Hair conditioning agents are specially suitable additives for shampoos. As such cationic polymers are particularly used, preferably in a proportion between 0.1 to 2, most preferrred from 0.25 to 1.25% by weight of the total composition.

European Patent Application No.337,354 already discloses the use of cationic polymers in combination with alkyl polyglucoside surfactants; the cationic polymers listed therein on pp. 3 to 7 are also suitable as conditioning additives in the compositions according to the invention.

Further conditioning additives are the known protein hydrolyzates, e.g., in a proportion of 0.25 to 5% by weight, preferably 0.5 to 2.5% by weight of the total composition.

Moreover, water-soluble collagen or water-soluble collagen derivatives are suitable.

Finally, as is already known, the various polysiloxanes may also be used as conditioning substances in the liquid body cleansing compositions according to the invention. Therein their preferred proportion is about 0.5 to about 5%, particularly 1 to 3% by weight of the total composition. Suitable are highly volatile as well as slightly volatile cyclic or linear polysiloxanes, e.g., silicone oils as known by their common names "Dimethicone" or "Phenyldimethicone" and "Cyclomethicone".

Also suitable for example are silicone derivatives as described in European Patent Application No.398,177 wherein they are used in combination with alkyl polyglucosides in liquid detergent compositions.

Whenever the composition according to the invention is a shampoo, it may also contain dyestuffs for direct or oxidative dyeing of the hair, i.e. so-called coloring or tinting shampoos.

The following examples illustrate the invention.

The preparation of the products according to the invention is performed by stirring together the single components in water, wherein pre-mixtures of various ingredients may also be used.

### Example 1: Shampoo for dry hair

| | |
|---|---|
| Disodium laurylether sulfosuccinate | 6.0% by wt. |
| Sodium C₁₄-C₁₆ olefin sulfonate | 5.0 |
| C₉-C₁₁-alkyl polyglucoside (P.G. =<1.4) | 5.0 |
| Lauryldimethyl amine oxide | 2.0 |
| Laurylhydroxysultaine | 0.5 |
| Triglycerol | 2.0 |
| Cationic polymer (Polyquaternium 7) | 2.1 |
| Glycerol caprate | 1.5 |
| Polyethylene glycol-60 sorbitane tristearate | 0.5 |
| Citric acid | 0.2 |
| Preservative (parabens) | 0.3 |
| Complexing agents | 0.2 |
| Perfume | 1.0 |
| Water | @ 100.0 |

In a comparison test, six volunteers with dry hair were treated with 5 different shampoos A, B, C, D and E which had the following combination:
- Shampoo A:: Composition according to the invention, as described in Example 1.
- Shampoo B:: Composition according to Example 1, wherein triglycerol was substituted by 2% by wt. of water.
- Shampoo C:: Composition according to Example 1, wherein triglycerol was substituted by 2% by wt. of glycerol.
- Shampoo D:: Composition according to Example 1, wherein triglycerol was substituted by 2% by weight of polyglycerol.
- Shampoo E:: Composition according to Example 1, wherein triglycerol was substituted by 2% by weight of diglycerol.

After the shampoo treatment the hair was dried and its state assessed for softness, volume (abundance) and gloss, according to a scale from 1 to 5 (1 = best, 5 = worst value) with the following average results:

| | |
|---|---|
| Shampoo A: | 2.08 |
| Shampoo B: | 3.83 |
| Shampoo C: | 3.58 |
| Shampoo D: | 3.17 |
| Shampoo E: | 2.33 |

As shown by this test, compositions containing triglycerol or diglycerol presented by far the best values.

### Example 2: Antidandruff shampoo

| | |
|---|---|
| Disodium laurylether sulfosuccinate | 11.0% by wt. |
| C₈-C₁₀-alkyl polyglucoside (P.G.: ∼ 1.5) | 5.0 |
| Lauryl hydroxysultaine | 3.0 |
| Triglycerol | 0.7 |
| Cationic polymer (Polyquaternium 7) | 0.3 |
| Sucrose laurate | 1.0 |
| Trideceth-8 | 0.6 |
| Citric acid | 0.1 |
| Preservatives (parabens) | 0.2 |
| Proctone olamine | 0.4 |
| Complexing agents | 0.2 |
| Perfume | 0.5 |
| Water | @ 100.0 |

This antidandruff shampoo also provided the hair with volume, gloss and softness, apart from its antidandruff performance.

### Example 3: Shampoo for fine hair

| | |
|---|---|
| Disodium laurylether sulfosuccinate | 8.00% by wt. |
| Caprylyl/capryl glucoside | 6.00 |
| Dimethyl lauryl amine oxide | 2.00 |
| Laurylhydroxysultaine | 1.00 |
| Sodium lauroyl sarcosinate | 2.00 |
| Sodium-PEG-6 coconut amide carboxylate | 1.50 |
| Triglycerol | 0.55 |
| Cationic polymer (Polyquaternium 7) | 0.55 |
| Polyethylene glycol-60 sorbitan tristearate | 1.00 |
| Citric acid | 0.35 |
| Complexing agents | 0.50 |
| Preservatives (parabens) | 0.25 |
| Perfume | 0.50 |
| Water | @ 100.00 |

Washing the hair with this shampoo resulted in soft and light hair presenting volume and gloss.

### Example 4: Shower gel

| | |
|---|---|
| Sodium laurylether sulfate | 20.0% by wt. |
| Lauryldimethyl amine oxide | 2.5 |
| Coconut fatty acid monoethanolamide | 2.5 |
| Coconut amidopropyl betaine | 3.0 |
| C₁₂-C₁₄-fatty acid monoglyceride | 2.5 |
| Triglycerol | 2.5 |
| Diglycerol | 3.0 |
| Perfume | 0.5 |
| Preservatives | 0.2 |
| Dyestuff | q.s. |
| Water | @ 100.00 |

After application and use of this shower bath, the skin feels pleasant and soft.

## Claims

1. Liquid aqueous personal cleansing composition containing about 5 to about 50% by weight, calculated to the total composition, of at least one anionic surfactant, characterized in that it comprises about 0.1 to about 10% by weight of triglycerol, calculated to the total composition.

2. Liquid personal cleansing composition according to claim 1, characterized in that it contains about 0.5 to 5% by weight of triglycerol, calculated to the total composition.

3. Liquid personal cleansing composition according to claim 1 or 2, characterized in that it additionally contains about 1 to about 15% by weight of at least one nonionic surfactant, calculated to the total composition.

4. Liquid personal cleansing composition according to claim 3, characterized in that it contains about 2.5 to about 10% by weight of an alkyl polyglucoside of the formula
R-O-(R¹O)ₙ-Zₓ,
wherein R denotes an alkyl group having 8 to 18 carbon atoms, R¹ an ethylene or propylene group, Z is a saccharide residue having 5 to 6 carbon atoms, n is a number from zero to ten, and x is a number between 1 and 2.5.

5. Use of about 0.1 to about 10% by weight, calculated to the total composition, of triglycerol and (or) diglycerol as conditioning agents in liquid aqueous personal cleansing compositions containing about 5 to about 50% by weight of at least one anionic surfactant, calculated to the total composition.

## Patentansprüche

1. Flüssiges wäßriges Körperreinigungsmittel auf Basis von etwa 5 bis etwa 50 Gew.-%, berechnet auf die Gesamtzusammensetzung, mindestens eines anionischen Tensids, dadurch gekennzeichnet, daß es etwa 0,1 bis etwa 10 Gew.-%, berechnet auf die Gesamtzusammensetzung, Triglycerin enthält.

2. Flüssiges Körperreinigungsmittel nach Anspruch 1, dadurch gekennzeichnet, daß es etwa 0,5 bis 5 Gew.-%, berechnet auf die Gesamtzusammensetzung, Triglycerin enthält.

3. Flüssiges Körperreinigungsmittel nach Anspruch 1 oder 2, dadurch gekennzeichnet, daß es zusätzlich etwa 1 bis etwa 15 Gew.-%, berechnet auf die Gesamtzusammensetzung, mindestens eines nichtionischen Tensids enthält.

4. Flüssiges Körperreinigungsmittel nach Anspruch 3, dadurch gekennzeichnet, daß es etwa 2,5 bis etwa 10 Gew.-% eines Alkylpolyglucosids der Formel
R-O-(R¹O)ₙ-Zₓ,
worin R eine Alkylgruppe mit 8 bis 18 Kohlenstoffatomen, R¹ eine Ethylen- oder Propylengruppe, Z einen Saccharidrest mit 5 bis 6 Kohlenstoffatomen, n eine Zahl von 0 bis 10 und x eine Zahl zwischen 1 und 2,5 bedeuten, enthält.

5. Verwendung von etwa 0,1 bis etwa 10 Gew.-%, berechnet auf die Gesamtzusammensetzung, an Triglycerin und/oder Diglycerin als Konditionierungsmittel in flüssigen wäßrigen Körperreinigungsmitteln auf Basis von etwa 5 bis etwa 50 Gew.-%, berechnet auf die Gesamtzusammensetzung, mindestens eines anionischen Tensids.

## Revendications

1. Composition nettoyante corporelle liquide aqueuse contenant d'environ 5 à environ 50 % en poids, par rapport à la composition totale, d'au moins un agent tensio-actif anionique, caractérisée en ce qu'elle comprend d'environ 0,1 à environ 10 % en poids de triglycérol, par rapport à la composition totale.

2. Composition nettoyante liquide corporelle selon la revendication 1, caractérisée en ce qu'elle contient d'environ 0,5 à 5 % en poids de triglycérol, par rapport à la composition totale.

3. Composition nettoyante liquide corporelle selon l'une des revendications 1 ou 2, caractérisée en ce qu'elle contient en outre d'environ 1 à environ 15 % en poids d'au moins un agent tensio-actif non ionique, par rapport à la composition totale.

4. Composition nettoyante liquide corporelle selon la revendication 3, caractérisée en ce qu'elle contient d'environ 2,5 à environ 10 % en poids d'un polyglucoside d'alkyle de formule
R-O-(R¹O)ₙ-Zₓ
dans laquelle R représente un groupe alkyle ayant de 8 à 18 atomes de carbone, R1 représente un groupe éthylène ou propylène, Z est un radical saccharide ayant de 5 à 6 atomes de carbone, n est un nombre de 1 à 10, et x est un nombre entre 1 et 2,5.

5. Utilisation d'environ 0,1 à environ 10 % en poids, par rapport à la composition totale, de triglycérol et (ou) de diglycérol comme agent de conditionnement dans des compositions nettoyantes corporelles liquides aqueuses contenant d'environ 5 à environ 50 % en poids d'au moins un agent tensio-actif anionique, par rapport à la composition totale.
